(19) Europäisches Patentamt
European Patent Office
Office européen des brevets

(11) **EP 4 113 483 A1**

(12) **EUROPEAN PATENT APPLICATION**
published in accordance with Art. 153(4) EPC

(43) Date of publication:
**04.01.2023 Bulletin 2023/01**

(21) Application number: **21759998.4**

(22) Date of filing: **01.03.2021**

(51) International Patent Classification (IPC):
**G09B 19/00** $^{(2006.01)}$   **A61B 3/113** $^{(2006.01)}$

(52) Cooperative Patent Classification (CPC):
**A61B 3/113; G09B 19/00**

(86) International application number:
**PCT/JP2021/007670**

(87) International publication number:
**WO 2021/172584 (02.09.2021 Gazette 2021/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.02.2020   JP 2020033697**
**08.01.2021   JP 2021002281**

(71) Applicant: **Daikin Industries, Ltd.**
**Osaka-shi, Osaka 530-8323 (JP)**

(72) Inventors:
• **KOBORI, Hirokazu**
  **Osaka-shi, Osaka 530-8323 (JP)**

• **NAKASHIMA, Yuta**
  **Suita-shi, Osaka 565-0871 (JP)**
• **IKEMOTO, Noriko**
  **Suita-shi, Osaka 565-0871 (JP)**
• **KIMURA, Tsukasa**
  **Suita-shi, Osaka 565-0871 (JP)**
• **NAGAHARA, Hajime**
  **Suita-shi, Osaka 565-0871 (JP)**
• **NUMAO, Masayuki**
  **Suita-shi, Osaka 565-0871 (JP)**
• **SHINOHARA, Kazumitsu**
  **Suita-shi, Osaka 565-0871 (JP)**

(74) Representative: **Global IP Europe**
**Patentanwaltskanzlei**
**Pfarrstraße 14**
**80538 München (DE)**

(54) **EFFICIENCY ESTIMATION DEVICE**

(57)     An efficiency inference apparatus (100) for inferring an efficiency of a subject (10) includes a biological information acquisition unit (20) and an inference unit (30). The biological information acquisition unit (20) acquires biological information of the subject (10). The inference unit (30) includes a trained model (40), and infers, using the trained model (40), the efficiency of the subject (10) from the biological information. The trained model (40) is a model trained with a training dataset including the biological information and the efficiency of the subject (10).

FIG. 1

EP 4 113 483 A1

**Description**

**TECHNICAL FIELD**

The present disclosure relates to an efficiency inference apparatus.

**BACKGROUND ART**

In the related art, a point of gaze of a subject onto a displayed object is identified, and a concentration level of the subject is inferred based only on data derived from a line of sight (PTL 1 (Japanese Unexamined Patent Application Publication No. 2016-224142)).

**SUMMARY OF INVENTION**

< Technical Problem >

In the case where a learning efficiency or a work efficiency of a subject is inferred, a concentration state that yields a higher productivity is desirably evaluated as a state of a higher concentration level. However, there is an issue that it is difficult to evaluate, based only on the data derived from the line of sight, whether the subject is in the concentration state that yields a higher productivity. Even if the line of sight is right at a target to be learned, the subject is not necessarily in the concentration state that yields a higher productivity. Thus, information taken into account by an inference model is insufficient. Further, as for a target to be inferred, it is desirable to infer an efficiency of a subject that serves as an evaluation indicator of the productivity, rather than a state of a living body such as the concentration state.

< Solution to Problem >

An efficiency inference apparatus according to a first aspect is an efficiency inference apparatus, for inferring an efficiency of a subject, including a biological information acquisition unit and an inference unit. The biological information acquisition unit acquires biological information of the subject. The inference unit infers the efficiency of the subject, based on the biological information.
This efficiency inference apparatus can infer the efficiency of the subject that serves as an evaluation indicator of the productivity, from the biological information of the subject.
An efficiency inference apparatus according to a second aspect is an efficiency inference apparatus, for inferring an efficiency of a subject, including a biological information acquisition unit and an inference unit. The biological information acquisition unit acquires biological information of the subject. The inference unit includes a trained model, and infers, using the trained model, the efficiency of the subject from the biological information. The trained model is a model trained by a training dataset including the biological information and the efficiency of the subject.
This efficiency inference apparatus can infer the efficiency of the subject that serves as an evaluation indicator of the productivity, from the biological information of the subject.
An efficiency inference apparatus according to a third aspect is the apparatus according to the first aspect or the second aspect, in which the efficiency is a learning efficiency of the subject.
An efficiency inference apparatus according to a fourth aspect is the apparatus according to the third aspect, in which the learning efficiency is based on a percentage of correct answers of the subject in a result of a test taken by the subject.
An efficiency inference apparatus according to a fifth aspect is the apparatus according to the third aspect, in which the learning efficiency is based on a time taken by the subject for answering in a result of a test taken by the subject.
An efficiency inference apparatus according to a sixth aspect is the apparatus according to the first aspect or the second aspect, in which the efficiency is a work efficiency of the subject.
This efficiency inference apparatus can infer the work efficiency of the subject.
An efficiency inference apparatus according to a seventh aspect is the apparatus according to the sixth aspect, in which the work efficiency is based on a work accuracy of the subject. The work accuracy of the subject includes a degree of occurrence of mistakes in a predetermined work of the subject.
An efficiency inference apparatus according to an eighth aspect is the apparatus according to the sixth aspect, in which the work efficiency is based on a work time taken for a predetermined work of the subject.
An efficiency inference apparatus according to a ninth aspect is the apparatus according to any of the first aspect to the eighth aspect, in which the biological information includes at least one of information related to a line of sight, a brain wave, a heartbeat, a body temperature, a body movement, a facial expression, or a face orientation of the subject.

An efficiency inference apparatus according to a tenth aspect is the apparatus according to any of the first aspect to the eighth aspect, in which the biological information is information obtained by processing a biological signal caused by a biological phenomenon or a biological activity and includes at least one of a drowsiness level, an arousal level, a concentration level, or a fatigue level.

An efficiency inference apparatus according to an eleventh aspect is the apparatus according to the tenth aspect, in which the biological signal caused by the biological phenomenon or the biological activity is a brain wave, a heartbeat, a body temperature, a body movement, a facial expression, or a face orientation of the subject.

An efficiency inference apparatus according to a twelfth aspect is the apparatus according to the ninth aspect, in which the information related to the line of sight of the subject includes at least one of a position of the line of sight, a movement of the line of sight, or a moving speed of the line of sight of the subject.

**BRIEF DESCRIPTION OF THE DRAWINGS**

Fig. 1 is a functional block diagram of an efficiency inference apparatus 100.
Fig. 2 is a diagram illustrating an example of a network architecture.
Fig. 3 is a diagram illustrating an example of learning data.
Fig. 4 is a diagram illustrating an example of a histogram of a learning efficiency.
Fig. 5 is a flowchart of the efficiency inference apparatus 100.
Fig. 6A is a diagram illustrating another example of the histogram of the learning efficiency.
Fig. 6B is a diagram illustrating another example of the histogram of the learning efficiency.
Fig. 7 is a functional block diagram of an efficiency inference apparatus 200.
Fig. 8 is a flowchart of the efficiency inference apparatus 200.

**DESCRIPTION OF EMBODIMENTS**

<First Embodiment>

(1) Overall Configuration of Efficiency Inference Apparatus

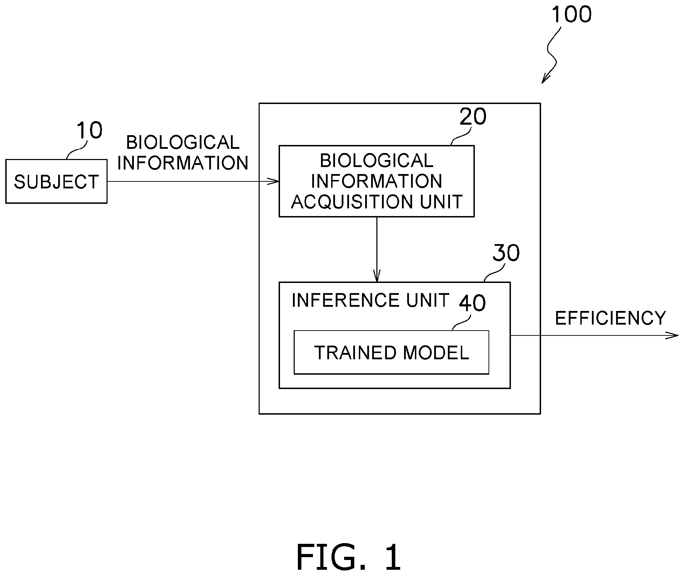

Fig. 1 illustrates an efficiency inference apparatus 100 according to the present embodiment. The efficiency inference apparatus 100 is implemented by a computer. The efficiency inference apparatus 100 includes a biological information acquisition unit 20 and an inference unit 30. The biological information acquisition unit 20 acquires biological information of a subject 10. The inference unit 30 infers, using a trained model 40, an efficiency of the subject 10 from the biological information of the subject 10.

(2) Detailed Configuration of Efficiency Inference Apparatus

(2-1) Biological Information Acquisition Unit

The biological information acquisition unit 20 acquires the biological information of the subject 10. The biological information is information related to a line of sight of the subject 10. The information related to the line of sight of the subject 10 includes at least one of a position of the line of sight, a movement of the line of sight, or a moving speed of the line of sight of the subject 10. In the present embodiment, the biological information acquisition unit 20 acquires, using a camera, the position of the line of sight of the subject 10.

(2-2) Inference Unit

The inference unit 30 includes the trained model 40, and infers, using the trained model 40, the efficiency of the subject 10 from the biological information. A processor such as a CPU or a GPU can be used as the inference unit 30. The inference unit 30 reads a program stored in a storage device (not illustrated) and performs predetermined arithmetic processing in accordance with this program. In accordance with the program, the efficiency inference apparatus 100 can further write the arithmetic operation result in the storage device or read information stored in the storage device. The storage device can be used as a database.

(2-3) Trained Model

(2-3-1) Network Architecture

The trained model 40 is a model trained with a training dataset including biological information and an efficiency of the subject 10.

In the present embodiment, to predict (infer) the efficiency of the subject 10 using the trained model 40, the line of sight of the subject 10 on a screen is used. Expression (1) below denotes a concatenation of positions $(x_K, y_K)^T$ on the screen.

$$p \in R^{2 \times K} \ (1)$$

For Expression (1), the number of positions of the line of sight of the subject 10 on the screen may vary from trial to trial. K denotes the number of collected pieces of line-of-sight coordinate data. For example, in the case where data is obtained for 10 seconds by a camera that acquires the line of sight of the subject 10 on the screen at one sample/second, then K = 10 holds.

A binary classification task is for finding a function that maps "p" in order to assign a label "efficient" or "inefficient".

In the present embodiment, a deep neural network is used. Specifically, a convolutional neural network having L convolutional layers (L = 1, 2, or 3) is used, and each layer is followed by, as an activation function, a ReLU function (ramp function) that is a non-linear function. An output from the highest convolutional layer is pooled by extracting a mean value or a maximum value to obtain a feature quantity in which a dimension in a time-series direction is compressed, and the pooling result is supplied to a fully connected layer having, as an activation function, a softmax function that is a non-linear function.

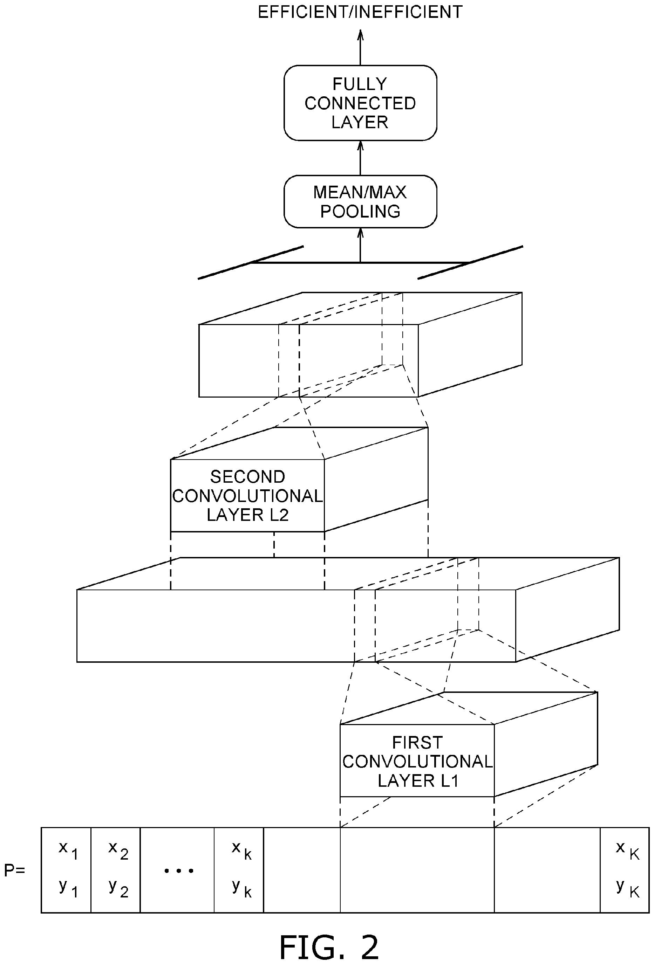

Fig. 2 illustrates an example of a network architecture including two convolutional layers.

First, as indicated by Expression (2) below, inputs to the network architecture are x-coordinate values and respective y-coordinate values on the screen.

$$p = (x_1, y_1), (x_2, y_2), \cdots (x_K, y_K) \ (2)$$

In Expression (2), $(x_K, y_K)$ denotes a vector. In the two-layer network architecture illustrated in Fig. 2, a plurality of input vectors $(x_1, y_1), (x_2, y_2) \cdots$ are input from an input side (lower side in Fig. 2), and an efficiency of the subject is output from an output side (upper side in Fig. 2). This network architecture is constituted by two layers L1 and L2.

The input vectors $(x_1, y_1), (x_2, y_2), \cdots (x_K, y_K)$ are input to the first convolutional layer L1. In the first convolutional layer L1, a feature quantity of the line of sight of the subject 10 is extracted, and a first feature quantity is output.

Then, the first feature quantity from the first convolutional layer L1 is input to the second convolutional layer L2. In the second convolutional layer L2, a feature quantity of the line of sight of the subject 10 is extracted from the first feature quantity, and a second feature quantity is output.

Then, the second feature quantity from the second convolutional layer L2 is input to a pooling layer (mean/max pooling). In the pooling layer, compression is performed through extraction of a mean value or a maximum value of the second feature quantity (feature map).

Then, the feature vector from the pooling layer is input to the fully connected layer. The output from the fully connected layer is input to an output layer (not illustrated). The output from the fully connected layer indicates whether the efficiency of the subject 10 is efficient or inefficient.

(3) Learning Process

The trained model 40 used by the efficiency inference apparatus 100 will be described. The trained model 40 uses an inference model obtained by training in advance a neural network with a training dataset including biological information and an efficiency of a subject.

In the learning process, a camera (not illustrated) of a personal computer for measuring information related to the line of sight and e-learning with a test are prepared. Training is performed using a training dataset in which the information related to the line of sight of the subject during e-learning is input data and information correlated to a percentage of correct answers of a test following the e-learning is output data.

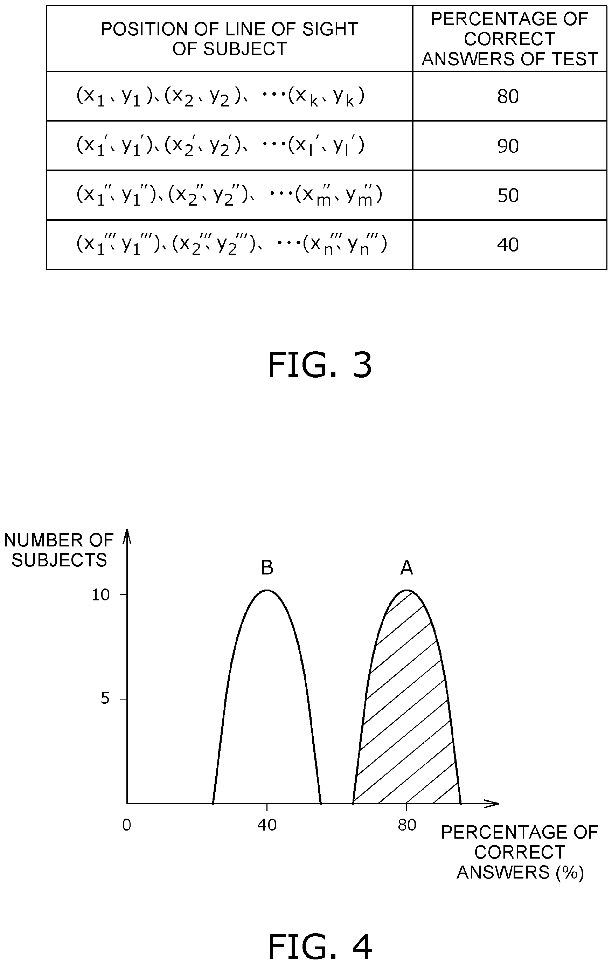

Fig. 3 illustrates an example of the learning data. In this example, positions where the line of sight of the subject 10 is located on the screen displaying the content during e-learning are recorded, and the recorded positions are used as the biological information of the subject. As illustrated in Fig. 3, in the case where the percentage of correct answers of the test following the e-learning is 80%, training is performed using a training dataset in which biological information in which the positions of the line of sight of the subject 10 are recorded as "$(x_1, y_1), (x_2, y_2), \cdots (x_K, y_K)$" is the input and the percentage of correct answers of 80% is the output. In addition, training is performed using a training dataset in which biological information in which the positions of the line of sight of the subject 10 are recorded as "$(x_1', y_1'), (x_2', y_2'), \cdots (x_l', y_l')$" is the input and the percentage of correct answers of 90% is the output. In addition, training is performed using a training dataset in which biological information in which the positions of the line of sight of the subject 10 are recorded as "$(x_1'', y_1''), (x_2'', y_2''), \cdots (x_m'', y_m'')$" is the input and the percentage of correct answers of 50% is the output. In addition, training is performed using a training dataset in which biological information in which the positions of the line of sight of the subject 10 are recorded as "$(x_1''', y_1'''), (x_2''', y_2'''), \cdots (x_{n'''}, y_{n'''})$" is the input and the percentage of correct answers of 40% is the output.

(4) Learning Efficiency

The efficiency of the subject 10 inferred by the efficiency inference apparatus 100 is a learning efficiency. The learning efficiency is based on the percentage of correct answers of the test taken by the subjects 10.

Fig. 4 illustrates an example of a histogram of the learning efficiency. The horizontal axis represents the percentage of correct answers of the test taken by the subjects 10, and the vertical axis represents the number of subjects. As illustrated in Fig. 4, the learning data is divided into two clusters, which are a cluster A and a cluster B. The cluster A has a peak at a point where the percentage of correct answers of the test taken by the subjects 10 is 80%. The cluster A is inferred to have a high learning efficiency because the percentage of correct answers of the test taken by the subjects 10 is high. The cluster B has a peak at a point where the percentage of correct answers of the test taken by the subjects 10 is 40%. The cluster B is inferred to have a low learning efficiency because the percentage of correct answers of the test taken by the subjects 10 is low.

(5) Overall Operation of Efficiency Inference Apparatus 100

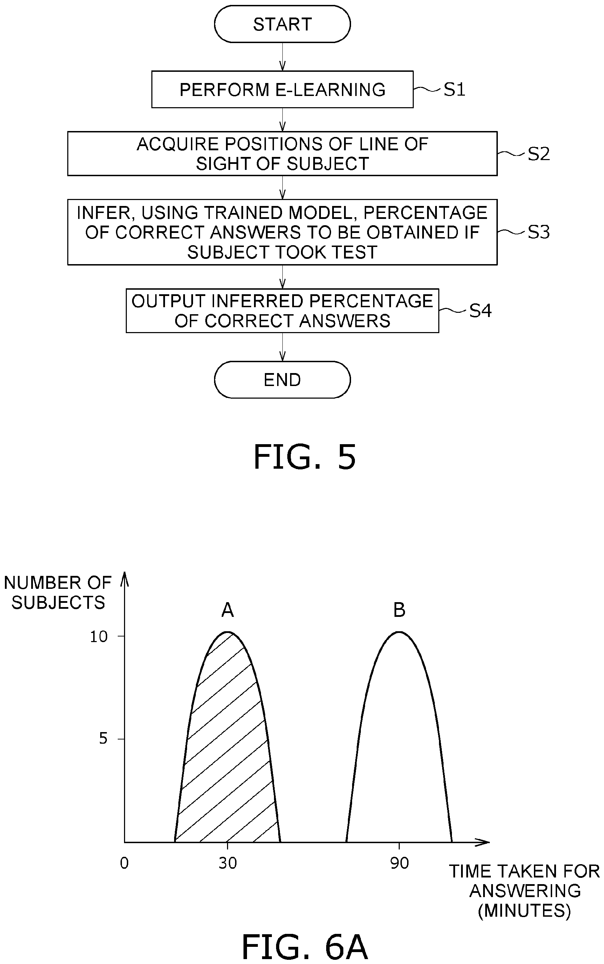

Fig. 5 is a flowchart of the efficiency inference apparatus 100. In the present embodiment, a case where the efficiency inference apparatus 100 is used in e-learning will be described.

First, in step S1, the subject 10 performs e-learning. An image of the subject 10 who is performing the e-learning is captured by a camera. The biological information acquisition unit 20 acquires the positions of the line of sight of the subject 10 who is performing the e-learning (step S2). From the positions of the line of sight of the subject 10, the inference unit 30 infers, using the trained model 40, a percentage of correct answers to be obtained if the subject 10 took the test (step S3). Then, the inference unit 30 outputs the percentage of correct answers inferred in step S3 to a display (not illustrated) (step S4). The display displays the inferred percentage of correct answers of the test.

(6) Features

(6-1)
The efficiency inference apparatus 100 according to the present embodiment is the efficiency inference apparatus 100, for inferring an efficiency of the subject 10, including the biological information acquisition unit 20 and the inference unit 30. The biological information acquisition unit 20 acquires biological information of the subject 10. The inference unit 30 includes the trained model 40, and infers, using the trained model 40, the efficiency of the subject 10 from the biological information. The trained model 40 is a model trained with a training dataset including biological information and an efficiency of the subject 10.

This efficiency inference apparatus 100 uses the trained model 40 trained with training data including the biological information and the efficiency of the subject 10. Thus, based on the biological information of the subject 10, the efficiency inference apparatus 100 can infer the efficiency of the subject 10 that serves as an evaluation indicator of the productivity.

(6-2)
In the efficiency inference apparatus 100 according to the present embodiment, the efficiency is a learning efficiency of the subject 10.

This efficiency inference apparatus 100 infers the learning efficiency of the subject 10, and thus can infer the learning efficiency of the subject 10 who performs e-learning.

(6-3)
In the efficiency inference apparatus 100 according to the present embodiment, the learning efficiency is based on a percentage of correct answers of the subject 10 in a result of a test taken by the subject 10.

In this efficiency inference apparatus 100, the learning efficiency is based on the percentage of correct answers of the subject 10 in the result of the test taken by the subject 10. Thus, content of the e-learning can be changed in accordance with the learning efficiency (the inferred percentage of correct answers) of the subject 10 who is performing the e-learning.

(6-4)

In the efficiency inference apparatus 100 according to the present embodiment, the information related to a line of sight of the subject includes at least one of a position of the line of sight, a movement of the line of sight, or a moving speed of the line of sight of the subject.

In this efficiency inference apparatus 100, various kinds of information related to the line of sight of the subject can be used as the biological information.

(7) Modifications

(7-1) Modification 1A

In the present embodiment, the case where the efficiency inference apparatus 100 infers the learning efficiency of the subject in e-learning has been described. However, the efficiency inference apparatus 100 may infer a learning efficiency in an online lecture.

This enables an online lecture suitable for a level of a student to be selected with reference to the learning efficiency.

(7-2) Modification 1B

In the present embodiment, the case where the efficiency inference apparatus 100 infers the learning efficiency of the subject in e-learning has been described. However, the efficiency inference apparatus 100 may infer a learning efficiency in a lecture at a tutoring school or at a school. In the case of inferring the learning efficiency in a lecture at a tutoring school or at a school, a model trained with a training dataset including information related to the line of sight of the entire class and a mean percentage of correct answers of the class is used as the trained model. Alternatively, as the trained model, a model trained with a training dataset including information related to a line of sight of a specific student and a percentage of correct answers of the student may be used.

(7-3) Modification 1C

In the present embodiment, the case where the biological information used in the efficiency inference apparatus 100 is information related to the line of sight of the subject 10 has been described. However, the biological information is not limited to this. The biological information used in the efficiency inference apparatus 100 may include at least one of a brain wave, a heartbeat, a body temperature, a body movement, a facial expression, or a face orientation of the subject. The biological information used in the efficiency inference apparatus 100 may be information obtained by processing a biological signal caused by a biological phenomenon or a biological activity, and may include at least one of a drowsiness level, an arousal level, a concentration level, or a fatigue level. Examples of the biological phenomenon include a heartbeat, a brain wave, a pulse, respiration, perspiration, and the like. In addition, examples of the biological activity include a facial expression, a face orientation, a body movement, and the like. The biological signal caused by the biological phenomenon and the biological activity includes a brain wave, a heartbeat, a body temperature, a body movement, a facial expression, a face orientation, or the like, and part of the biological signal caused by the biological phenomenon is referred to as a vital sign.

In the efficiency inference apparatus according to a modification 1C, the biological information includes at least one of information related to a line of sight, a brain wave, a heartbeat, a body temperature, a body movement, a facial expression, or a face orientation of the subject. Thus, the efficiency inference apparatus according to the modification 1C can infer the efficiency of the subject from the various kinds of biological information of the subject. In addition, in the efficiency inference apparatus according to the modification 1C, the biological information is information obtained by processing a biological signal caused by a biological phenomenon or a biological activity, and includes at least one of a drowsiness level, an arousal level, a concentration level, or a fatigue level. Thus, the efficiency inference apparatus according to the modification 1C can infer the efficiency of the subject using, as the biological information, in addition to a biological signal caused by a biological phenomenon or a biological activity in a living body, the information obtained by processing the biological signal caused by the biological phenomenon or the biological activity.

In the present embodiment, the case where the biological information acquisition unit 20 acquires the biological information of the subject from a camera has been described. However, the biological information acquisition unit 20 may acquire the biological information of the subject from a biological sensor.

(7-4) Modification 1D

In the present embodiment, as for the efficiency inferred by the efficiency inference apparatus 100, the case where the learning efficiency is based on a percentage of correct answers of a test taken by the subject 10 has been described. However, the learning efficiency may be based on a time taken by the subject 10 for answering the test or a time spent by the subject 10 for learning.

For example, as illustrated in Fig. 6A, the case where the time taken for answering the test is short corresponds to a cluster A having a peak at a point where the time taken for answering is 30 minutes. In this case, the learning efficiency is inferred to be high. In addition, the case where the time taken for answering the test is long corresponds to a cluster B having a peak at a point where the time taken for answering is 90 minutes. In this case, the learning efficiency is inferred to be low.

In the efficiency inference apparatus according to a modification 1D, the learning efficiency is based on the time taken by the subject for answering in a result of a test taken by the subject. Thus, content of the e-learning can be changed in accordance with the time taken for answering the test taken by the subject after the e-learning.

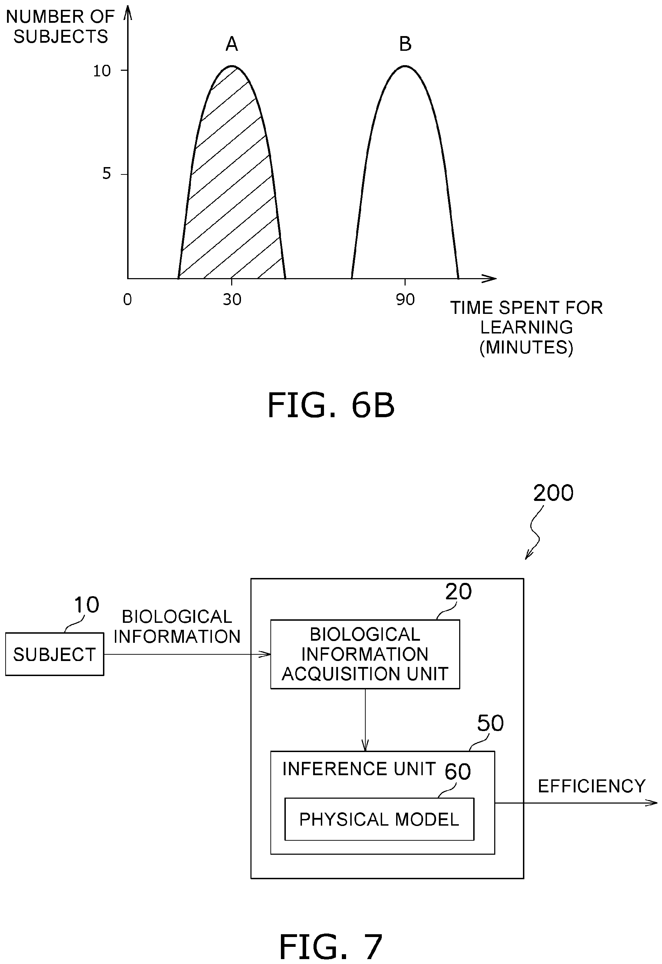

In addition, as illustrated in Fig. 6B, the case where the time spent for learning is short corresponds to a cluster A having a peak at a point where the time spent for learning is 30 minutes. In this case, the learning efficiency is inferred to be high. In addition, the case where the time spent for learning is long corresponds to a cluster B having a peak at a point where the time spent for learning is 90 minutes. In this case, the learning efficiency is inferred to be low. The time spent for learning is a time taken to learn materials when the e-learning consists of the materials and the test.

In the efficiency inference apparatus according to the modification 1D, the learning efficiency is based on a time spent by the subject for learning in a result of a test taken by the subject. Thus, content of the e-learning can be changed in accordance with the time spent for learning by the subject who has performed the e-learning.

The time spent for learning may be a playback time when learning is performed in an online lecture. For example, if the subject rewinds the content many times when performing learning in the online lecture, the playback time of the online lecture increases.

In addition, for the learning efficiency, any two or all of the percentage of correct answers of the test, the time taken for answering the test, and the time spent for learning of the subject 10 may be used as the indicators. The time taken by the subject for answering the test or the time spent by the subject for learning is acquired using a timer.

(7-5) Modification 1E

In the present embodiment, the case where the efficiency inferred by the efficiency inference apparatus 100 is the learning efficiency of the subject 10 has been described. However, the efficiency inferred by the efficiency inference apparatus 100 may be a work efficiency of the subject.

The work efficiency of the subject is based on a work accuracy of the subject. The work accuracy of the subject includes a degree of occurrence of mistakes in a predetermined work of the subject. The work efficiency of the subject may be based on a work time taken for the predetermined work of the subject. The predetermined work of the subject is a work at an assembly line at a factory, a data input work, or the like.

The efficiency inference apparatus according to a modification 1E infers the work efficiency of the subject, and thus can infer the work efficiency of the subject at an assembly line at a factory, a data input work, or the like.

In addition, in the efficiency inference apparatus according to the modification 1E, the work efficiency is based on the work accuracy of the subject, and the work accuracy of the subject includes the degree of occurrence of mistakes in the predetermined work of the subject. Thus, assignment to a work place suitable for each person at the assembly line, a guidance for improving erroneous inputs of the subject who has performed the data input work, or the like can be performed.

In addition, in the efficiency inference apparatus according to the modification 1E, the work efficiency includes the work time taken for the predetermined work of the subject. Thus, a load variation at the assembly line can be improved or the subject who has performed the data input work can be prompted to take a break.

<Second Embodiment>

(1) Overall Configuration of Efficiency Inference Apparatus

Fig. 7 illustrates an efficiency inference apparatus 200 according to the present embodiment. The efficiency inference apparatus 200 is implemented by a computer. The efficiency inference apparatus 200 includes a biological information acquisition unit 20 and an inference unit 50. The biological information acquisition unit 20 acquires biological information of a subject 10. The inference unit 50 infers, using a physical model 60, an efficiency of the subject 10 from the biological information of the subject 10.

(2) Detailed Configuration of Efficiency Inference Apparatus

(2-1) Biological Information Acquisition Unit

The biological information acquisition unit 20 acquires the biological information of the subject 10. The biological information is information related to a line of sight of the subject 10. The information related to the line of sight of the subject 10 includes at least one of a position of the line of sight, a movement of the line of sight, or a moving speed of the line of sight of the subject 10. In the present embodiment, the biological information acquisition unit 20 acquires, using a camera, the position of the line of sight of the subject 10.

(2-2) Inference Unit

The inference unit 50 includes the physical model 60, and infers, using the physical model 60, the efficiency of the subject 10 from the biological information. A processor such as a CPU or a GPU can be used as the inference unit 50. The inference unit 50 reads a program stored in a storage device (not illustrated) and performs predetermined arithmetic processing in accordance with this program. In accordance with the program, the efficiency inference apparatus 200 can further write the arithmetic operation result in the storage device or read information stored in the storage device. The storage device can be used as a database.

(2-3) Physical Model

The physical model 60 used by the efficiency inference apparatus 200 will be described. The physical model 60 indicates a correlation between biological information and an efficiency of the subject 10.
In the present embodiment, to predict (infer) the efficiency of the subject 10 using the physical model 60, the line of sight of the subject 10 on a screen is used. Positions where the line of sight of the subject 10 is located on the screen displaying the content during e-learning are acquired, and the acquired positions are used as the biological information of the subject.
Suppose that the acquired biological information in which the positions of the line of sight of the subject 10 are "$(x_1, y_1), (x_2, y_2), \cdots (x_k, y_k)$" is input. In this case, a percentage of correct answers of the test taken after e-learning is obtained using the physical model 60 of Expression (3) below.

$$\text{Percentage of Correct Answers} = f(x_1, x_2, \cdots x_k, y_1, y_2, \cdots y_k) \ (3)$$

(3) Overall Operation of Efficiency Inference Apparatus 200

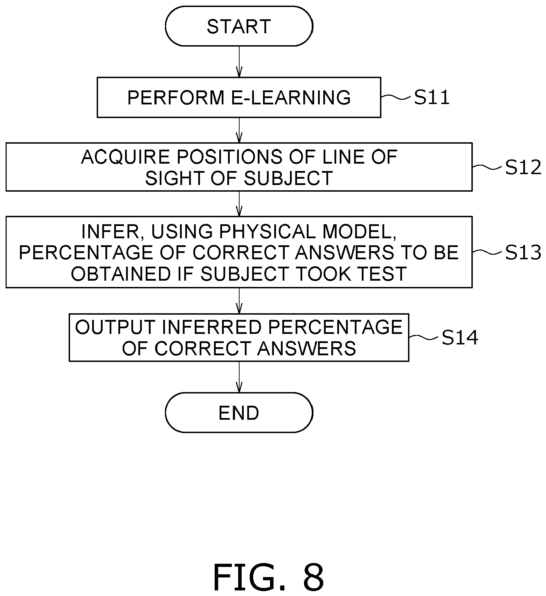

Fig. 8 is a flowchart of the efficiency inference apparatus 200. In the present embodiment, a case where the efficiency inference apparatus 200 is used in e-learning will be described.
First, in step S11, the subject 10 performs e-learning. An image of the subject 10 who is performing the e-learning is captured by a camera. The biological information acquisition unit 20 acquires the positions of the line of sight of the subject 10 who is performing the e-learning (step S12). From the positions of the line of sight of the subject 10, the inference unit 50 infers, using the physical model 60, a percentage of correct answers to be obtained if the subject 10 took the test (step S13). Then, the inference unit 50 outputs the percentage of correct answers inferred in step S13 to a display (not illustrated) (step S14). The display displays the inferred percentage of correct answers of the test.

(4) Features

(4-1)
The efficiency inference apparatus 200 according to the present embodiment is the efficiency inference apparatus 200, for inferring an efficiency of the subject 10, including the biological information acquisition unit 20 and the inference unit 50. The biological information acquisition unit 20 acquires biological information of the subject 10. The inference unit 50 infers the efficiency of the subject 10, based on the biological information.
In this efficiency inference apparatus 200, the physical model 60 is based on a correlation between biological information and an efficiency of the subject 10. Thus, based on the biological information of the subject 10, the efficiency inference apparatus 200 can infer the efficiency of the subject 10 that serves as an evaluation indicator of the productivity.

(5) Modifications

(5-1) Modification 2A

In the present embodiment, the case where the efficiency inference apparatus 200 infers the learning efficiency of the subject in e-learning has been described. However, the efficiency inference apparatus 200 may infer a learning efficiency in an online lecture.

(5-2) Modification 2B

In the present embodiment, the case where the efficiency inference apparatus 200 infers the learning efficiency of the subject in e-learning has been described. However, the efficiency inference apparatus 200 may infer a learning efficiency in a lecture at a tutoring school or at a school.

(5-3) Modification 2C

In the present embodiment, the case where the biological information used in the efficiency inference apparatus 200 is information related to the line of sight of the subject 10 has been described. However, the biological information is not limited to this. The biological information used in the efficiency inference apparatus 200 may include at least one of a brain wave, a heartbeat, a body temperature, a body movement, a facial expression, or a face orientation of the subject. The biological information used in the efficiency inference apparatus 200 may include information obtained by processing a biological signal caused by a biological phenomenon or a biological activity, for example, converted information such as a drowsiness level, an arousal level, a concentration level, or a fatigue level.
In the present embodiment, the case where the biological information acquisition unit 20 acquires the biological information of the subject from a camera has been described. However, the biological information acquisition unit 20 may acquire the biological information of the subject from a biological sensor.

(5-4) Modification 2D

In the present embodiment, as for the efficiency inferred by the efficiency inference apparatus 200, the case where the learning efficiency is based on a percentage of correct answers of a test taken by the subject 10 has been described. However, the learning efficiency may be based on a time taken by the subject 10 for answering the test or a time spent by the subject 10 for learning.

(5-5) Modification 2E

In the present embodiment, the case where the efficiency inferred by the efficiency inference apparatus 200 is the learning efficiency of the subject 10 has been described. However, the efficiency inferred by the efficiency inference apparatus 200 may be a work efficiency of the subject.

(5-6) Modification 2F

While the embodiments of the present disclosure have been described above, it should be understood that various modifications can be made on the configurations and details without departing from the gist and the scope of the present disclosure that are described in the claims.

**REFERENCE SIGNS LIST**

| | |
|---|---|
| 10 | subject |
| 20 | biological information acquisition unit |
| 30, 50 | inference unit |
| 40 | trained model |
| 60 | physical model |
| 100, 200 | efficiency inference apparatus |

## EP 4 113 483 A1

CITATION LIST

PATENT LITERATURE

PTL 1: Japanese Unexamined Patent Application Publication No. 2016-224142

Claims

1.  An efficiency inference apparatus for inferring an efficiency of a subject, comprising:

    a biological information acquisition unit configured to acquire biological information of the subject; and
    an inference unit configured to infer the efficiency of the subject, based on the biological information.

2.  An efficiency inference apparatus (100) for inferring an efficiency of a subject (10), comprising:

    a biological information acquisition unit (20) configured to acquire biological information of the subject; and
    an inference unit (30) including a trained model (40) and configured to infer, using the trained model, the efficiency of the subject from the biological information, wherein
    the trained model is a model trained with a training dataset including the biological information and the efficiency of the subject.

3.  The efficiency inference apparatus according to Claim 1 or 2, wherein
    the efficiency is a learning efficiency of the subject.

4.  The efficiency inference apparatus according to Claim 3, wherein
    the learning efficiency is based on a percentage of correct answers of the subject in a result of a test taken by the subject.

5.  The efficiency inference apparatus according to Claim 3, wherein
    the learning efficiency is based on a time taken by the subject for answering in a result of a test taken by the subject.

6.  The efficiency inference apparatus according to Claim 1 or 2, wherein
    the efficiency is a work efficiency of the subject.

7.  The efficiency inference apparatus according to Claim 6, wherein
    the work efficiency is based on a work accuracy of the subject, and
    the work accuracy of the subject includes a degree of occurrence of mistakes in a predetermined work of the subject.

8.  The efficiency inference apparatus according to Claim 6, wherein
    the work efficiency is based on a work time taken for a predetermined work of the subject.

9.  The efficiency inference apparatus according to any of Claims 1 to 8, wherein
    the biological information includes at least one of information related to a line of sight, a brain wave, a heartbeat, a body temperature, a body movement, a facial expression, or a face orientation of the subject.

10. The efficiency inference apparatus according to any of Claims 1 to 8, wherein
    the biological information is information obtained by processing a biological signal caused by a biological phenomenon or a biological activity and includes at least one of a drowsiness level, an arousal level, a concentration level, or a fatigue level.

11. The efficiency inference apparatus according to Claim 10, wherein
    the biological signal caused by the biological phenomenon or the biological activity is a brain wave, a heartbeat, a body temperature, a body movement, a facial expression, or a face orientation of the subject.

12. The efficiency inference apparatus according to Claim 9, wherein
    the information related to the line of sight of the subject includes at least one of a position of the line of sight, a movement of the line of sight, or a moving speed of the line of sight of the subject.

FIG. 1

EFFICIENT/INEFFICIENT

FULLY
CONNECTED
LAYER

MEAN/MAX
POOLING

SECOND
CONVOLUTIONAL
LAYER L2

FIRST
CONVOLUTIONAL
LAYER L1

$$P = \begin{array}{|c|c|c|c|c|c|c|} \hline x_1 & x_2 & & x_k & & & x_K \\ y_1 & y_2 & \cdots & y_k & & & y_K \\ \hline \end{array}$$

# FIG. 2

| POSITION OF LINE OF SIGHT OF SUBJECT | PERCENTAGE OF CORRECT ANSWERS OF TEST |
|---|---|
| $(x_1 、 y_1)、(x_2 、 y_2)、 \cdots (x_k 、 y_k)$ | 80 |
| $(x_1' 、 y_1')、(x_2' 、 y_2')、 \cdots (x_l' 、 y_l')$ | 90 |
| $(x_1'' 、 y_1'')、(x_2'' 、 y_2'')、 \cdots (x_m'' 、 y_m'')$ | 50 |
| $(x_1''' 、 y_1''')、(x_2''' 、 y_2''')、 \cdots (x_n''' 、 y_n''')$ | 40 |

# FIG. 3

# FIG. 4

START

PERFORM E-LEARNING — S1

ACQUIRE POSITIONS OF LINE OF
SIGHT OF SUBJECT — S2

INFER, USING TRAINED MODEL, PERCENTAGE
OF CORRECT ANSWERS TO BE OBTAINED IF — S3
SUBJECT TOOK TEST

OUTPUT INFERRED PERCENTAGE
OF CORRECT ANSWERS — S4

END

# FIG. 5

NUMBER OF
SUBJECTS

A          B

10

5

0          30          90          TIME TAKEN FOR
                                     ANSWERING
                                     (MINUTES)

# FIG. 6A

FIG. 6B

FIG. 7

START

PERFORM E-LEARNING ⟩～S11

ACQUIRE POSITIONS OF LINE OF
SIGHT OF SUBJECT ⟩～S12

INFER, USING PHYSICAL MODEL,
PERCENTAGE OF CORRECT ANSWERS TO BE
OBTAINED IF SUBJECT TOOK TEST ⟩～S13

OUTPUT INFERRED PERCENTAGE
OF CORRECT ANSWERS ⟩～S14

END

# FIG. 8

<div align="center">

**INTERNATIONAL SEARCH REPORT**

</div>

| | International application No. |
|---|---|
| | PCT/JP2021/007670 |

**A. CLASSIFICATION OF SUBJECT MATTER**
G09B 19/00(2006.01)i; A61B 3/113(2006.01)i
FI: G09B19/00 G; A61B3/113

According to International Patent Classification (IPC) or to both national classification and IPC

**B. FIELDS SEARCHED**

Minimum documentation searched (classification system followed by classification symbols)
G09B1/00-G09B9/56,G09B17/00-G09B19/26,G06Q10/00-G06Q10/10,G06Q30/00-G06Q30/08,G06Q50/00-G06Q50/20, G06Q50/26-G06Q99/00

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus (JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>Y | US 2019/0080623 A1 (MASSACHUSETTS INSTITUTE OF TECHNOLOGY) 14 March 2019 (2019-03-14) paragraphs [0032]-[0035] | 1-4, 6-7, 9-10, 12<br>5, 8, 11 |
| Y | JP 2019-128365 A (BENESSE CORPORATION) 01 August 2019 (2019-08-01) paragraph [0096] | 5, 8, 11 |
| Y | JP 2016-224142 A (FUJITSU LTD.) 28 December 2016 (2016-12-28) paragraph [0009] | 11 |

☐ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 April 2021 (16.04.2021) | 11 May 2021 (11.05.2021) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

International application No.

PCT/JP2021/007670

| Patent Documents referred in the Report | Publication Date | Patent Family | Publication Date |
|---|---|---|---|
| US 2019/0080623 A1 | 14 Mar. 2019 | US 2019/0079917 A1 | |
| JP 2019-128365 A | 01 Aug. 2019 | (Family: none) | |
| JP 2016-224142 A | 28 Dec. 2016 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016224142 A